# EUROPÄISCHE PATENTSCHRIFT

(11) **EP 0 872 735 B1**
(45) Veröffentlichungstag und Bekanntmachung des Hinweises auf die Patenterteilung: **22.09.2004**
(21) Anmeldenummer: 98106611.1
(22) Anmeldetag: 09.04.1998
(51) Int. Cl.: G01N 33/543, G01N 33/551, G01N 33/547, G01N 21/55

(54) **Verfahren zum Aufbringen von Reagenzspots**
Process for the application of reagent spots
Méthode d'application de tâches d'agents

(30) Priorität: 14.04.1997 DE 19715484
(43) Veröffentlichungstag der Anmeldung: 21.10.1998
(73) Patentinhaber: Biacore AB, 754 50 Uppsala (SE)
(72) Erfinder: Sluka, Peter, Dr., 82362 Weilheim (DE); Knoll, Wolfgang, Prof. Dr., 55124 Mainz (DE); Zizlsperger, Manfred, 55126 Mainz (DE)
(74) Vertreter: Weiss, Wolfgang, Dipl.-Chem. Dr.

(56) Entgegenhaltungen:
- EP-A- 0 664 452
- DE-A- 4 039 677

## Beschreibung

Die Erfindung betrifft ein Verfahren zum Aufbringen von räumlich definierten Reagenzflächen bzw. Reagenzspots auf eine Festphase mit einer Metall- oder Metalloxidoberfläche sowie eine Festphasenbindematrix, welche unterschiedliche Festphasenreaktanten oder/und Festphasenreaktanten in unterschiedlichen Konzentrationen in räumlich definierten Reagenzspots umfaßt.

Es ist eine Reihe von Verfahren bekannt, mit denen kleine Reagenzmengen auf unterschiedliche Oberflächen, wie etwa Glas- oder Kunststoffträger aufgebracht werden können. Solche Verfahren führen zu einer Mikrostrukturierung der Reagenzien auf der Oberfläche, z. B. in Form von räumlich voneinander getrennten Reagenzspots. Von besonderem Interesse sind dabei mikrostrukturierte Oberflächen, bei denen die Reagenzspots jeweils unterschiedliche Funktionalitäten aufweisen, sogenannte Arrays, bei denen die einzelnen Reagenzspots z. B. unterschiedliche Reaktanten wie etwa DNA-Fragmente oder Antikörper umfassen. In WO 92/10 092 ist ein Verfahren beschrieben, mit dem über photoreaktive Verbindungen und Bestrahlung durch Masken eine Vielzahl verschiedener Strukturen auf einem Glasträger erzeugt werden kann. In US-4,877,745 ist ein Verfahren beschrieben, bei dem mittels Ink-Jet (Tintenstrahl) auf Kunststoffträgern unterschiedlich funktionalisierte Spots aufgebracht werden können.

Im Gegensatz zu Kunststoffoberflächen weisen Metall- und Metalloxidoberflächen den Vorteil auf, daß sie durch Selbstassemblierungstechniken mit einer genau definierten Matrixschicht belegt werden können. Eine selbstassemblierende Monoschicht (SAM) bildet sich z.B. bei der Adsorption von organischen Alkylthiolen auf einer Goldoberfläche aus, wobei die spontane Organisation einer solchen dichtgepackten Monoschicht auf starken spezifischen Wechselwirkungen zwischen dem Trägermaterial und dem Adsorbens beruhen (Nuzzo et al., J.Am.Chem.Soc. 105 (1983) 4481). Auf diese Weise kann eine genau definierte Monoschicht einer Bindematrix auf die Oberfläche von Metallen, wie z. B. Gold oder Silber, aufgebracht werden. Die spezifische Bindefähigkeit von selbstassemblierten Festphasen kann zudem durch Verdünnung des spezifischen Festphasenreaktanten weiter optimiert werden, wie in EP-A-0 515 615 beschrieben.

Auch die Beschichtung von Metalloberflächen mit Mikrostrukturen auf Basis von selbstassemblierten Monolagen ist bekannt. So beschreiben Whitesides et al., Langmuir 10 (1994) 1498-1511 ein Verfahren, bei dem Reagenzien mittels spezieller, mikrostrukturierter Silikonstempel auf eine Edelmetalloberfläche aufgestempelt werden. Dadurch ist eine Erzeugung von mikrostrukturierten Monolagen mit räumlich voneinander getrennten Bereichen möglich. Die einzelnen Bereiche werden bei einem solchen Stempelverfahren allerdings alle identisch funktionalisiert erhalten, d. h. eine unterschiedliche Funktionalität durch unterschiedliche Belegung einzelner Spots, wie bei einer Array-Struktur, kann mit dieser Technik nicht erhalten werden.

Weiterhin ist es bekannt, Mikrostrukturen von selbstassemblierten Monoschichten auf Edelmetalloberflächen durch Bestrahlen von vollflächig mit Thiolen beschichteten Substraten durch Masken und anschließendem Waschen zu bilden (Hemminger et al., Langmuir 10 (1994), 626-628). Auch bei diesem Verfahren werden räumlich getrennte Bereiche gebildet, die alle identisch funktionalisiert sind. Eine weitere Möglichkeit zur Herstellung von Reagenzspots besteht darin, zunächst auf einen Träger bereits räumlich voneinander getrennte Goldspots aufzubringen, die dann nachträglich mit Reagenzien belegt werden. Die Erzeugung solcher räumlich getrennten Goldspots ist jedoch aufwendig und muß durch Bedampfen des Substrats über Masken erzeugt werden. Zudem erfordert das nachträgliche Belegen der Goldspots mit Reagenzien, z. B. mit Mikropipettoren, punktgenaues Arbeiten, was bei Strukturen im µm-Bereich nur schwierig und mit hohem technischem Aufwand zu realisieren ist.

Die Aufgabe der Erfindung bestand deshalb darin, ein Verfahren bereitzustellen, mit dem auf einfache Weise Array-Strukturen von Reagenzspots auf Metall- oder Metalloxidoberflächen aufgebracht werden können.

Diese Aufgabe wird erfindungsgemäß durch ein Verfahren zum Aufbringen von räumlichen definierten Reagenzflächen auf eine Festphase gelöst, welches dadurch gekennzeichnet ist, daß eine ein adsorptionsfähiges Bindereagenz enthaltende Flüssigkeit im Bereich von räumlich definierten Flächen mit einer Festphase, die eine im wesentlichen kontinuierliche Metall- oder Metalloxidoberfläche umfaßt für eine ausreichende Zeitdauer, um die Ausbildung adsorptiver Bindungen zwischen Bindereagenz und Festphase zu ermöglichen, in Kontakt gebracht wird.

Überraschenderweise konnten die im Stand der Technik auftretenden Nachteile dadurch beseitigt werden, daß die zur Selbstassemblierung verwendeten Reagenzien direkt in einer Lösung auf einen vollflächig mit einem Metall oder einem Metalloxid beschichteten Reagenzträger spotweise aufgebracht werden. Das Aufbringen der Reagenzspots kann z. B. mittels Ink-Jet-Verfahren (Tintenstrahlverfahren) oder mit einer automatischen Mikropipettiervorrichtung erfolgen. Die Reagenzlösung wird dabei in Form kleiner Tröpfchen, bevorzugt in Form von Mikrotröpfchen auf die Oberfläche aufgebracht.

Nach dem Aufbringen der Reagenzlösung wird die Oberfläche für eine gewisse Zeitdauer, z.B. 1 bis 60 min, bevorzugt 5 bis 10 Minuten inkubiert, um die Ausbildung von adsorptiven Bindungen zwischen Bindereagenz und Metall- oder Metalloxidoberfläche zu ermöglichen. Die Inkubationszeitdauer hängt von dem verwendeten Bindereagenz und von der Oberfläche ab, wobei lange genug inkubiert wird, um die Ausbildung von adsorptiven Bindungen zu ermöglichen. Dabei wird eine selbstassemblierte Monolage des Bindereagenzes auf der Metalloder Metalloxidoberfläche in Form von räumlich abgegrenzten Flächen gebildet. Nach dem Inkubieren wird die Oberfläche schnell mit einem großen Lösungsmittelüberschuß gewaschen, um ein Verschmieren der voneinander angrenzenden Flächen zu vermeiden. Als Lösungsmittel können je nach verwendetem Bindereagenz wässrige oder/und organische Lösungsmittel verwendet werden.

Überraschenderweise ist es möglich, durch Aufbringen von adsorptiv bindenden Reagenzien auf eine durchgehende Metall- oder Metalloxidschicht in Form von Spots räumlich abgegrenzte Flächen zu bilden, wobei weder während des Aufbringens noch danach ein signifikantes Verschmieren benachbarter Reagenzspots auftritt. Es werden trotz der lediglich adsorptiven Bindung zwischen Oberfläche und Bindereagenz diskrete Spots gebildet, da das adsorptionsfähige Bindereagenz überraschend ortsfest ist. Ein wesentlicher Vorteil des Verfahrens besteht darin, daß auf einfache Weise Oberflächenregionen mit unterschiedlichen Belegungen oder Funktionalitäten hergestellt werden können, die für Multiparameterassays in Nachweisverfahren, z.B. in immunologischen oder Nukleinsäurehybridisierungsassays eingesetzt werden können. Weiterhin wurde festgestellt, daß das Bindereagenz nach Abschluß der Selbstassemblierung durch den Waschvorgang nicht über den Träger in andere Reagenzspots verschleppt wird. Weiterhin wurde festgestellt, daß auch nach Fertigstellung der funktionalisierten Oberflächen die einzelnen räumlich abgegrenzten Flächenbereiche stabil sind, d.h. die adsorbierten Bindereagenzien bleiben am selben Ort und wandern nicht zu unbelegten Stellen auf der Metall- oder Metalloxidoberfläche.

Die Flächen der aufgebrachten Reagenzspots weisen bevorzugt einen Durchmesser von ≤ 5 mm, besonders bevorzugt einen Durchmesser von ≤ 1 mm auf. Am meisten bevorzugt werden Reagenzspots im Mikrometerbereich, z.B. mit einem Durchmesser von 50 bis 500 µm aufgebracht. Dies entspricht einem aufgetropften Flüssigkeitsvolumen der Reagenzlösung von etwa 0,1 bis 10 nl. Da mit dem erfindungsgemäßen Verfahren räumlich scharf begrenzte Spots erhalten werden, ist es möglich eine Mikroarraystruktur zu erzeugen. Im Bereich der einzelnen Spots können jeweils unterschiedliche Reaktanten oder/und unterschiedliche Reaktantkonzentrationen, z. B. für immunologische Test oder Nukleinsäure-Hybridisierungstests aufgebracht werden.

Bevorzugte Oberfläche ist eine Edelmetalloberfläche, besonders bevorzugt eine Gold- oder Silberoberfläche und am meisten bevozugt eine Goldoberfläche. Eine solche Metalloberfläche kann beispielsweise durch Aufdampfen einer dünnen Metallschicht auf einen Träger, beispielsweise einen Glasträger gebildet werden. Eine solche aufgedampfte Schicht ist bevorzugt 10 bis 100 nm dick. Bei der Verwendung einer Edelmetalloberfläche wird als adsorptionsfähiges Bindereagenz vorzugsweise ein SH- oder SS-Reagenz verwendet. Solche Thiol- bzw. Disulfidreagenzien sind z.B. in der DE 40 39 677 ausführlich beschrieben. Besonders bevorzugt wird als adsorptionsfähiges Bindereagenz eine Thiol- oder Disulfidgruppe enthaltende Verbindung verwendet, welche zudem eine spezifisch bindefähige Gruppe, wie z.B. eine Antigen-, Hapten- oder eine Biotingruppierung enthält. Als SAM an eine Oberfläche adsorbierte biotinylierte Thiolreagenzien können anschließend mit Streptavidin belegt werden, wobei ein unterschiedlicher Biotingehalt in verschiedenen Spots zu verschiedenen Streptavidinschichten führt.

Es ist aber auch möglich, ein Metalloxid als Oberfläche zu verwenden. Beispiele für geeignete Metalloxidoberflächen sind SiO₂, TiO₂, Al₂O₃, Sn₂O₃, Ag₂O, La₂O₃, Ta₂O₅ und Gemische davon. Bevorzugt wird eine Metalloxidoberfläche aus SiO₂ und/oder TiO₂ verwendet. Wenn eine Metalloxidoberfläche verwendet wird, wird als adsorptionsfähiges Bindereagenz bevorzugt eine eine Silangruppe enthaltende Verbindung verwendet. Solche Verbindungen sind beispielsweise in EP-A-0 664 452 beschrieben. Bei der Adsorption von Silangruppen enthaltenden Verbindungen an Metalloxidoberflächen findet nach der Adsorption eine kovalente Vernetzung, beispielsweise durch Erhitzen statt.

Die erfindungsgemäß verwendeten adsorptions fähigen Bindereagenzien enthalten neben der jeweiligen adsorptionsfähigen Gruppe, wie etwa eine SH-, SS- oder Silangruppe, bevorzugt wenigstens eine spezifisch bindefähige Gruppe, wie etwa Biotin, ein Biotinderivat, Streptavidin, ein Hapten, ein Antigen und/oder eine Nukleinsäuresequenz, welche spezifisch mit einem Bindepartner, z.B. einem nachzuweisenden Analyten binden. Der Nachweis der Bindung von Analyten an eine erfindungsgemäße, funktionalisierte Festphasenmatrix kann beispielsweise durch konfokale Scannerfluoreszenzmikroskopie oder durch Plasmonenresonanzspektroskopie erfolgen. Bevorzugt wird der Nachweis mittels Plasmonenresonanzmikroskopie (B.Rothenhäusler et al., Nature, Vol. 332 (1988) 615-617) durchgeführt. Mit der Oberflächenplasmonenresonanz kann die Bindung des Analyten dabei ohne die Verwendung von Markierungsreagenzien nachgewiesen werden. Besonders bevorzugt wird zum Nachweis der Bindung eines Analyten die gleichzeitige Bestimmung von Plasmonenresonanz und Fluoreszenzdetektion verwendet. Hierbei werden unabhängig voneinander das Plasmonenresonanz- und das Fluoreszenzsignal erhalten, wodurch neben einer hohen Sensitivität (10⁻¹⁴ mol/l) auch die Bestimmung von Reaktionskinetiken möglich ist.

Ein weiterer Gegenstand der Erfindung ist eine Festphasenmatrix umfassend einen Träger, welcher im wesentlichen kontinuierlich mit einer Metall- oder Metalloxidschicht überzogen ist, auf der räumlich definierte Reagenzflächen angeordnet sind, welche (a) unterschiedliche Festphasenreaktanten enthalten oder/und (b) einen Festphasenreaktanten in unterschiedlichen Konzentrationen enthalten. Bevorzugt umfaßt eine solche Festphasenbindematrix mindestens drei unterschiedlich funktionalisierte Bereiche auf einer Oberfläche, besonders bevorzugt mindestens 5 unterschiedlich funktionalisierte Bereiche. Die Durchmesser der Reagenzspots betragen bevorzugt ≤ 5 mm, mehr bevorzugt ≤ 1 mm und am meisten bevorzugt ≤ 500 µm. Eine solche Festphasenmatrix mit Reagenzspots im µm-Bereich kann auch als Mikroarray bezeichnet werden.

Ein weiterer Gegenstand der Erfindung ist ein Verfahren zum Nachweis einer Bindung oder/und Wechselwirkung von Analyten, wobei eine erfindungsgemäße Festphasematrix eingesetzt wird. Bei der Verwendung der Plasmonenresonanzspektroskopie oder/und Plasmonenresonanzmikroskopie als Detektionsverfahren ist dabei eine Echtzeitdetektion möglich, wobei eine Markierung nicht erforderlich ist, da die Schichtdicke gemessen werden kann. Ein solches Verfahren kann in der molekularen Diagnostik zur Bestimmung von Allergien, in der Immunologie sowie zur Nukleinsäure-Hybridisierung verwendet werden. Besonders bevorzugt verwendet man zum Nachweis der Bindung oder/und Wechselwirkung des Analyten eine Kombination von Plasmonenresonanzspektroskopie und Fluoreszenzdetektion oder eine Kombination von Plasmonenresonanzmikroskopie und Fluoreszenzdetektion.

Die Erfindung wird durch die beigefügten Zeichnungen und die folgenden Beispiele weiter erläutert, worin
- Figur 1: das Prinzip des Aufbringens der Reagenzspots veranschaulicht,
- Figur 2: Reagenzien, welche zur Bildung von selbstassemblierten Monoschichten geeignet sind, darstellt,
- Figur 3: den experimentellen Aufbau eines Oberflächenplasmonenmikroskops zeigt,
- Figur 4: mit einem Plasmonen-Mikroskop erhaltene Aufnahmen von erfindungsgemäß auf eine Goldoberfläche aufgebrachten Reagenzspots zeigt und
- Figur 5: die zeitabhängige Reflexionsintensität gegen die Zeit einer Matrix mit vier verschiedenen hydrophilen Thiolgemischen, erhalten durch Oberflächenplasmonenmikroskopie darstellt.

### Beispiel 1

### Aufbringen von Reagenzspots auf eine Oberfläche

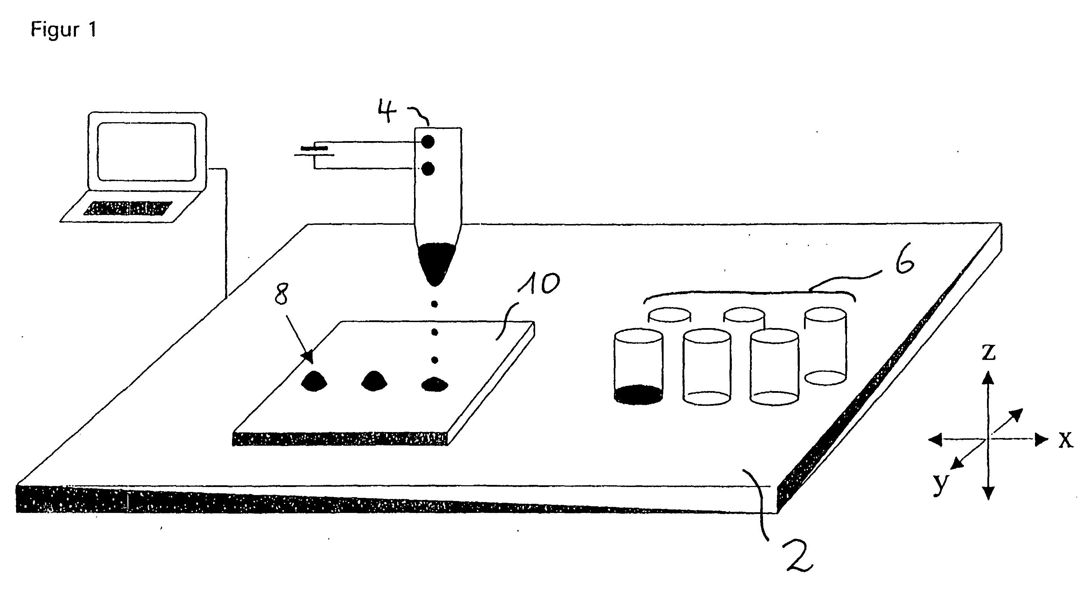

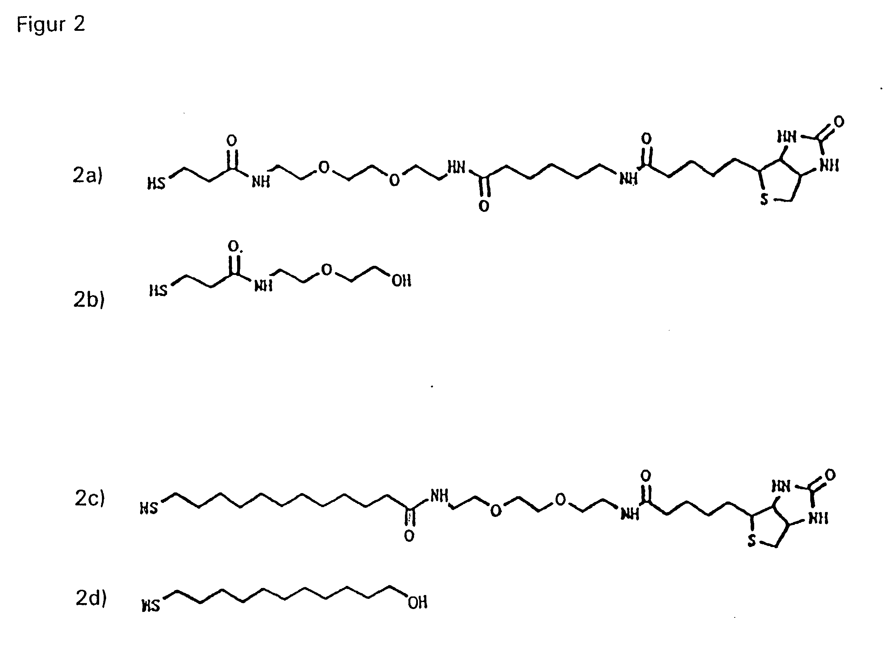

Das Prinzip des erfindungsgemäßen Verfahrens wird anhand einer bevorzugten Ausführungsform unter Bezugnahme auf Figur 1 erläutert. Zunächst wird mit Hilfe einer Hochvakuum-Beschichtungsanlage (Fa. Leibold) eine ca. 50 nm dicke Goldschicht auf einen Glasträger aus LASFN9 Hochindexglas im Hochvakuum (ca. 10⁻⁷ mbar) aufgedampft. Unmittelbar nach dem Aufdampfen der Goldschicht wird der Träger (10) auf einem XYZ-Tisch (Isel-EP 1090/4) (2) eingespannt, welcher mit einer Silizium-Piezopumpe (GeSIM, Dresden) (4) versehen ist. Auf der Arbeitsfläche des XYZ-Tisches (2) ist außerdem eine Mikrotiterplatte (6) befestigt, welche die verschiedenen, aufzubringenden adsorptionsfähigen Bindereagenzien gelöst in einem Lösungsmittel, sowie die reinen Lösungsmittel in jeweils verschiedenen Vertiefungen enthält. Als Substanzen für die Adsorption wurden ein wasserlösliches Thiolsystem, bestehend aus HS-Prop-DADOO-X-Biotin (bindefähiges Reagenz) (Fig. 2a) und HS-C2-Aminoethoxyethanol (Verdünnerkomponente) (Fig. 2b) in einer Konzentration von 5 x 10⁻⁴ molar in Wasser und ein ethanollösliches Thiolsystem, bestehend aus HS-C12-DADOO-Biotin (bindefähige Biotinkomponente) (Fig. 2c) und Mercaptoundecanol (Verdünnerkomponente) (Fig. 2d) in einer Konzentration von 5 x 10⁻⁴ molar in Ethanol gelöst. In unterschiedlichen Vertiefungen der Mikrotiterplatte wurde diese Systeme in unterschiedlichen Mischungsverhältnissen mit einem Anteil der Biotinkomponente von 0 %, 10 %, 50 % bzw. 100 % vorgelegt. Zum Aufbringen von Reagenzspots auf die Goldoberfläche wird nun die Spitze der Pumpe (4) in die entsprechende Flüssigkeit eingetaucht, dann zur programmierten Position auf der goldbeschichteten Oberfläche gefahren und dort wird ein Tropfen mit einem Volumen von ca. 1 nl (8) abgesetzt. Danach wird die Pumpe zweimal mit reinem Lösungsmittel gespült und der Vorgang mit der nächsten Lösung wiederholt, wobei der nächste Spot einem Abstand von ca. 600 µm zum vorhergehenden Spot abgesetzt wird. Dieser Vorgang kann beliebig oft wiederholt werden.

Anschließend wird die Goldoberfläche, auf die die Reagenzspots aufgetragen wurden, ca. 10 min setzen gelassen, wobei sich auf der mit den Tropfen belegten Oberfläche eine selbstassemblierte Monoschicht ausbildet. Anschließend wird die Goldoberfläche ganzflächig mit reinem Lösungsmittel gespült, um nicht adsorbierte Moleküle abzuwaschen. Auf diese Weise können innerhalb kurzer Zeit Oberflächen mit räumlich abgegrenzten Reaktionsspots erzeugt werden. Der Durchmesser der Spots beträgt ca. 200 µm.

### Beispiel 2

### Plasmonenmikroskopische Untersuchung der auf eine Goldoberfläche aufgebrachten Reagenzspots

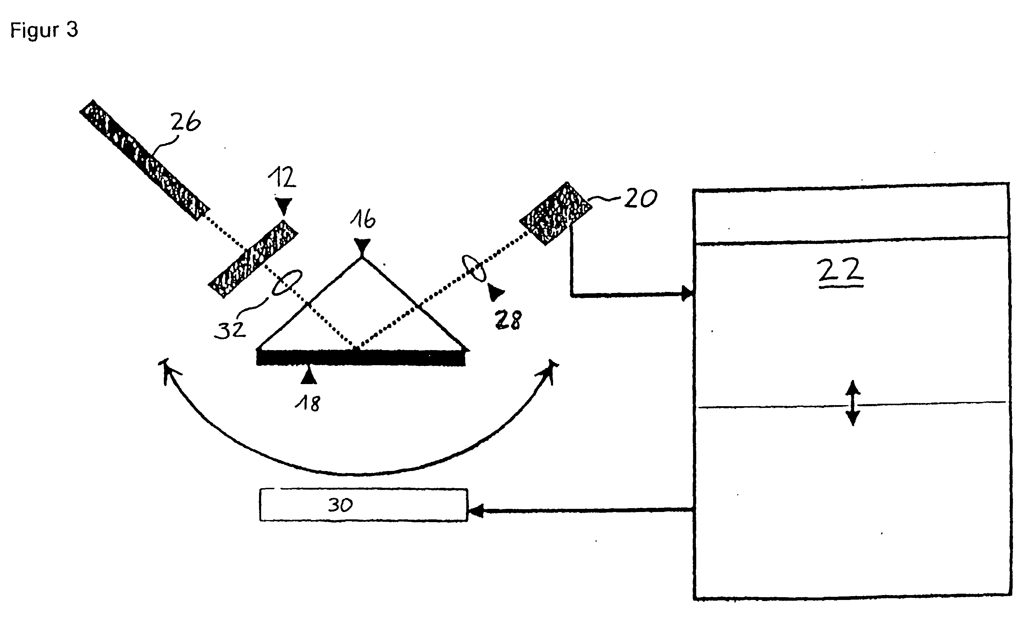

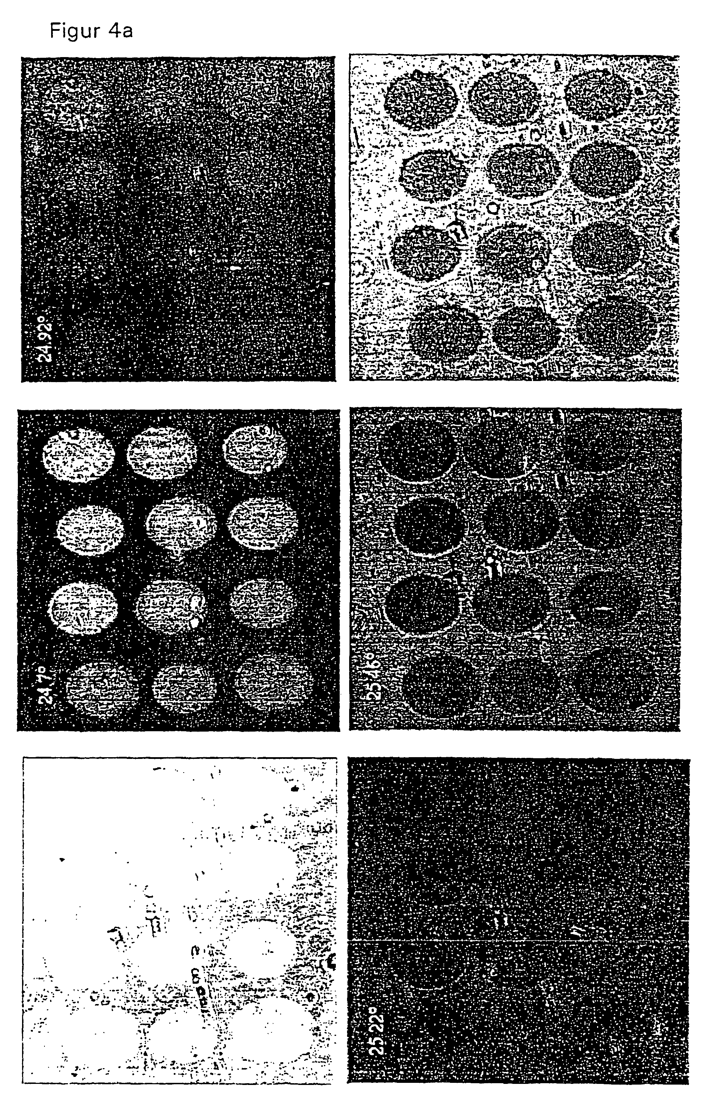

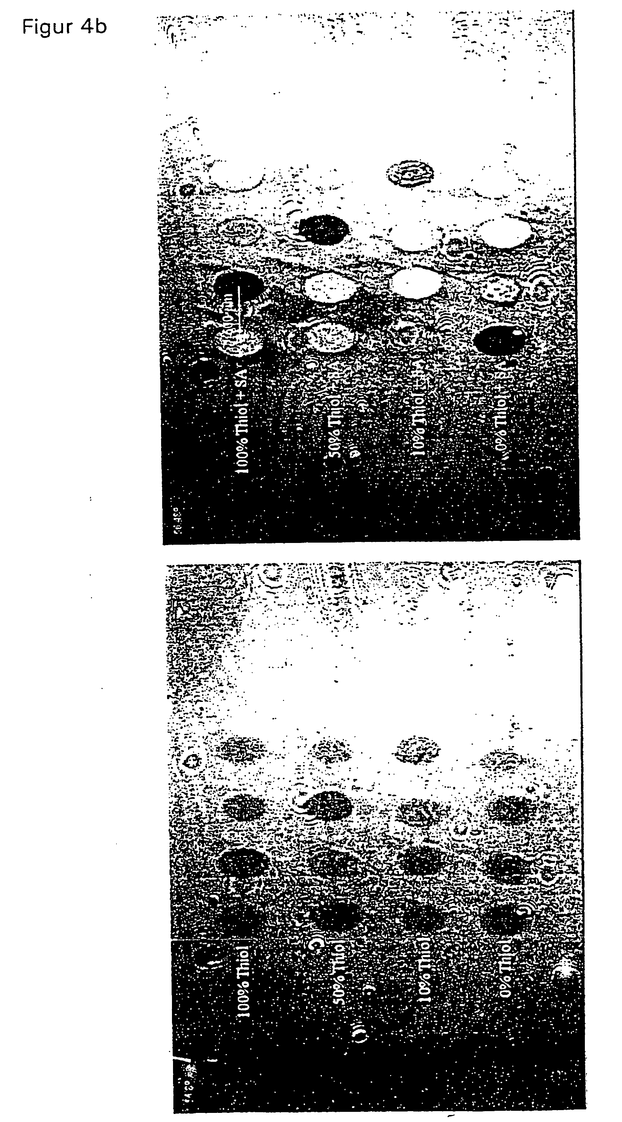

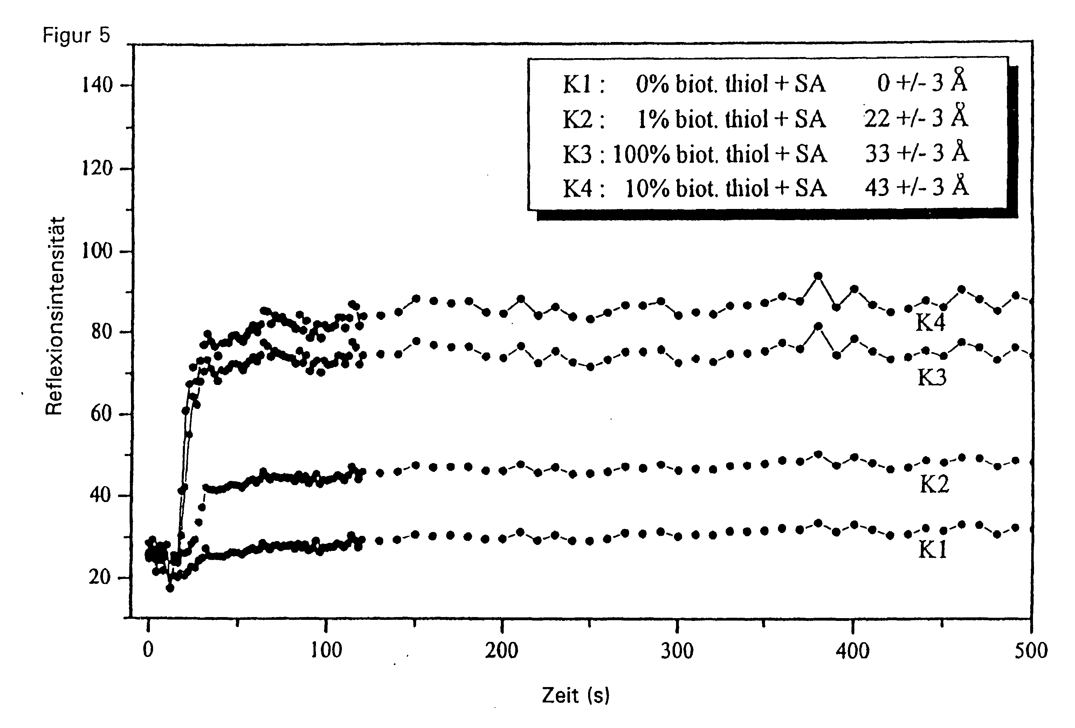

Zur Bestimmung der optischen Schichtdicken der präparierten Biotin-Monoschichten und zur Untersuchung des Bindeverhaltens gegenüber Streptavidin wurde die mit Reagenzspots präparierte Goldoberfläche mit Hilfe der Oberflächenplasmonenmikroskopie in der Kretschmannkonfiguration (prismengekoppelt) untersucht. Dabei werden, wie in Figur 3 dargestellt, mit durch einen Polarisator (12) p-polarisiertem aufgeweitetem Laserlicht (roter He-Ne-Laser (14) durch ein Prisma (16) aus hochbrechendem Glas bei einem spezifischen Einfallswinkel auf der Metallschicht (18) Oberflächenplasmonen angeregt. Das reflektierte Licht wird in Abhängigkeit vom Einfallswinkel von einer CCD-Kamera (20) aufgezeichnet. Weiterhin umfaßt der in Figur 3 dargestellte Aufbau ein Goniometer (30) sowie zwei Linsen (28, 32) zur Bildung einer Mikroskopoptik. Unterschiedliche Schichtdicken auf den Reagenzspots resultieren in unterschiedlichen Hell-Dunkel-Kontrasten auf dem aufgezeichneten Bild. Mit Hilfe von Bildauswertesystemen (22) umfassend einen Computer, kann der absolute Schichtdickenzuwachs sowie dessen zeitlicher Verlauf mit einer Genauigkeit von ca. 0,1 nm bestimmt werden. Figur 4 zeigt die mittels CCD-Kamera erhaltenen Bilder der mit Thiol beschichteten Spots vor und nach Streptavidinanbindung. In Figur 5 ist der zeitliche Verlauf der über die Bildauswertung erhaltenen Intensitäten einer Streptavidinanbindung an unterschiedlich beladene Spots dargestellt. In Figur 4a sind sechs Aufnahmen zu sehen, welchejeweils zwölf gleich funktionalisierte Thiol-Spots bei verschiedenen Einfallswinkel des Laserlichts zeigen. In Figur 4b ist ein mit 16 Reagenzspots beschichteter Träger zu sehen, wobei jeweils 4 der Spots die gleiche Funktionalisierung aufweisen, wobei das Bild links vor der Bindung von Streptavidin, das Bild rechts nach der Bindung von Streptavidin aufgenommen ist. Wie deutlich zu sehen ist, ist es mit dem erfindungsgemäßen Verfahren möglich, räumlich getrennte, unterschiedlich funktionalisierte Reagenzspots auf eine durchgängige Oberfläche aufzubringen.

Figur 5 stellt den zeitlichen Verlauf der Adsorption von Streptavidin an eine mit vier unterschiedlichen hydrophilen Thiol-Gemischen belegte Oberfläche, erhalten durch die Auswertung der Reflexionsintensität der Plasmonenresonanzmikroskopie, dar. K1 stellt einen Reagenzspot dar, auf den eine reine Lösungsmittellösung inkubiert wurde. Dieser Spot wurde anschließend mit Streptavidin behandelt. Wie in Figur 5 zu sehen ist, findet nur eine sehr geringe Bindung von Streptavidin an den Reagenzspot statt. K2 stellt einen Reagenzspot dar, der mit einer Lösung bestehend aus 1 % biotinyliertem Thiol und 99 % Verdünnerthiol inkubiert und anschließend mit Streptavidin behandelt wurde. Es ist eine deutliche höhere Streptavidinbindung als bei der Kurve K1 zu erkennen. K3 stellt einen Reagenzspot dar, der mit 100% biotinyliertem Thiol, also ohne Verdünnermoleküle inkubiert wurde und anschließend mit Streptavidin behandelt wurde. Es ergibt sich eine deutliche Erhöhung der Schichtdicke auf 33 ± 3 Å im Verhältnis zu K2, wo die Schichtdicke 22 ± 3 Å betrug. K4 schließlich stellt einen Reagenzspot dar, welcher mit 10 % biotinyliertem Thiol und 90% Verdünnermolekülen behandelt wurde. Dabei bildet sich eine verdünnte homogene Bindeschicht aus, welche nach Behandlung mit Streptavidin die höchste Reflexion aufweist, woraus eine Schichtdicke von 43 ± 3 Angström berechnet werden kann. Wie aus Figur 6 zu sehen ist, können auf einen einzigen flächigen Goldträger Reagenzspots mit unterschiedlicher Belegung aufgebracht werden, wodurch ein Array-System gebildet wird. Weiterhin ist es möglich, unter Verwendung eines solchen Arrays Bindungen oder/und Wechselwirkungen von Analyten nachzuweisen, wobei sowohl die Schichtdicke (Belegungsmenge) als auch die Reaktionskinetik (Adsorptionsgeschwindigkeit) bestimmt werden kann.

## Patentansprüche

1. Verfahren zum Aufbringen von räumlich definierten Reagenzflächen auf eine Festphase,
**dadurch gekennzeichnet,**
**dass** eine ein adsorptionsfähiges Bindereagenz enthaltende Flüssigkeit im Bereich von räumlich definierten Flächen mit einer Festphase, die eine kontinuierliche Metall- oder Metalloxidoberfläche umfassf, für eine ausreichende Zeitdauer, um die Ausbildung adsorptiver Bindungen zwischen Bindereagenz und Festphase zu ermöglichen, in Kontakt gebracht wird, wobei die Reagenzflächen
(a) jeweils unterschiedliche Festphasenreaktanten enthalten oder/und
(b) Festphasenreaktanten in jeweils unterschiedlichen Konzentrationen enthalten.

2. Verfahren nach Anspruch 1,
**dadurch gekennzeichnet,**
**dass** eine Metalloberfläche verwendet wird.

3. Verfahren nach Anspruch 2,
**dadurch gekennzeichnet,**
**dass** als adsorptionsfähiges Bindereagenz eine eine Thiol- oder Disulfidgruppe enthaltende Verbindung verwendet wird.

4. Verfahren nach Anspruch 1,
**dadurch gekennzeichnet,**
**dass** eine Metalloxidoberfläche verwendet wird.

5. Verfahren nach Anspruch 4,
**dadurch gekennzeichnet,**
**dass** als adsorptionsfähiges Bindereagenz eine eine Silangruppe enthaltende Verbindung verwendet wird.

6. Verfahren nach einem der vorhergehenden Ansprüche,
**dadurch gekennzeichnet,**
**dass** das adsorptionsfähige Bindereagenz in Form einer selbstassemblierenden Monoschicht aufgebracht wird.

7. Festphasenmatrix umfassend einen Träger, welcher kontinuierlich mit einer Metall- oder Metalloxidschicht überzogen ist, auf der räumlich definierte Reagenzflächen angeordnet sind, welche
(a) jeweils unterschiedliche Festphasenreaktanten enthalten oder/und
(b) einen Festphasenreaktanten in jeweils unterschiedlichen Konzentrationen enthalten.

8. Verfahren zum Nachweis einer Bindung oder/und Wechselwirkung von Analyten,
**dadurch gekennzeichnet,**
**dass** man eine Festphasenmatrix nach Anspruch 7 verwendet und die Bindung oder/und Wechselwirkung des Analyten auf an sich bekannte Weise nachweist.

## Claims

1. Process for the application of spatially defined reagent areas to a solid phase
**characterized in that**
a liquid containing an absorptive binding reagent is contacted with spatially defined areas of a solid phase which comprises a continuous metal or metal oxide surface for an adequate time period to enable the formation of absorptive bonds between binding reagent and solid phase, where the reagent areas
(a) each contain different solid phase reactants or/and
(b) each contain a solid phase reactant at different concentrations.

2. Process as claimed in claim 1,
**characterized in that**
a metal surface is used.

3. Process as claimed in claim: . 2,
**characterized in that**
a compound containing a thiol or disulphide group is used as the adsorptive binding reagent.

4. Process as claimed in claim 1,
**characterized in that**
a metal oxide surface is used.

5. Process as claimed in claim 4,
**characterized in that**
a compound containing a silane group is used as the adsorptive binding reagent.

6. Process as claimed in one of the previous claims,
**characterized in that**
the adsorptive binding reagent is applied in the form of a self-assembled monolayer.

7. Solid phase matrix comprising a support which is continuously coated with a metal or metal oxide layer on which spatially defined reagent areas are arranged which
(a) each contain different solid phase reactants or/and
(b) each contain a solid phase reactant at different concentrations.

8. Method for the detection of a binding or/and interaction of analytes,
**characterized in that**
a solid phase matrix as claimed in claim 7 is used and the binding or/and interaction of the analyte is detected in a well-known manner.

## Revendications

1. Procédé pour le dépôt de spots de réactif définis dans l'espace sur une phase solide,
**caractérisé en ce que**,
un liquide contenant un réactif de liaison adsorbant est mis en contact, dans la zone de spots définis dans l'espace, avec une phase solide qui a une surface essentiellement continue de métal ou d'oxyde métallique, pour une durée suffisante pour permettre la formation de liaisons adsorbantes entre le réactif de liaison et la phase solide, les spots de réactif
(a) contenant chacun différents réactants de phase solide et/ou
(b) contenant des réactants de phase solide en différentes concentrations.

2. Procédé selon la revendication 1,
**caractérisé en ce que**, on utilise une surface métallique.

3. Procédé selon la revendication 2,
**caractérisé en ce que**
on utilise, comme réactif de liaison adsorbant, un composé contenant un groupe thiol ou disulfure.

4. Procédé selon la revendication 1,
**caractérisé en ce que**
on utilise une surface d'oxyde métallique.

5. Procédé selon la revendication 4,
**caractérisé en ce que**
on utilise comme réactif de liaison adsorbant, un composé contenant un groupe silane.

6. Procédé selon l'une quelconque des revendications précédentes,
**caractérisé en ce que**
on applique le réactif de liaison adsorbant en forme de monocouche auto-assemblante.

7. Matrice de phase solide comprenant un support qui est essentiellement recouvert en continu d'une couche de métal ou d'oxyde métallique sur laquelle sont disposés, des spots de réactif définis dans l'espace lesquels
(a) contiennent chacun différents réactants de phase solide et/ou
(b) contiennent des réactants de phase solide en différentes concentrations.

8. Procédé de détection d'une liaison et/ou interaction d'analytes,
**caractérisé en ce que**
on utilise une matrice de phase solide selon la revendication 7 et on détecte la liaison et/ou l'interaction de l'analyte d'une manière connue en soi.
